# EUROPEAN PATENT APPLICATION

(11) **EP 2 196 928 A1**
(43) Date of publication of application: **16.06.2010**
(21) Application number: 09177289.7
(22) Date of filing: 27.11.2009
(51) Int. Cl.: G06F 19/00, A61B 5/00, A61B 5/0402

(54) **Method and system for configuring a monitoring device**

(30) Priority: 10.12.2008 US 331626
(71) Applicant: General Electric Company, Schenectady, NY 12345 (US)
(72) Inventor: Kohls, Mark Robert, New Berlin, WI 53151 (US); Peterson, James Russel, Fond du Lac, WI 54935 (US)
(74) Representative: Illingworth-Law, William Illingworth

(57) **Abstract**

A system (6) for configuring monitoring devices (10a-10n) in a clinical study is disclosed herein. The system (6) includes a configuration apparatus (8) adapted to receive and retain configuration data, and a plurality of patient monitoring devices (l0a-10n) that are each independently connectable to the configuration apparatus (8). The patient monitoring devices (10a-10n) are adapted to facilitate the performance of a clinical study. The configuration apparatus (8) is adapted to selectively transmit the configuration data to each of the patient monitoring devices (10a-10n) so that each of the patient monitoring devices (10a-10n) is generally identically configured in a manner that minimizes labor requirements.

## Description

### BACKGROUND OF THE INVENTION

The subject matter disclosed herein relates to a method and system for configuring a monitoring device that is adapted for use in a clinical study.

One method for assessing the safety of a new medication involves the performance of a clinical study. Patient monitoring devices are commonly implemented during the course of the clinical study to help evaluate the new medication. As an example, an electrocardiograph device may be implemented to monitor the patients to whom the new medication has been administered, and to thereby help evaluate the safety of the new medication. Patient monitoring may, for example, comprise the acquisition of multiple diagnostic readings over time (e.g., acquiring a 10S resting ECG once a month over three years as is typically done in late phase trials); or to the acquisition of monitoring data during a single diagnostic reading (e.g., continuously recording 48 hrs of ECG data as is typically done in phase I trials).

Large-scale clinical studies may implement one or more patient monitoring devices at each of several hundred different evaluation sites located all over the world. It has generally been necessary to manually configure each of the monitoring devices. It is typically the case that the configuration requirements are common to each of the patient monitoring devices in order to maintain uniformity and provide more accurate clinical study results.

One problem is that it is labor intensive to manually configure each patient monitoring device to be implemented in the clinical study. Another problem is that the process of manually configuring and programming the patient monitoring devices is subject to human error.

### BRIEF DESCRIPTION OF THE INVENTION

The above-mentioned shortcomings, disadvantages and problems are addressed herein which will be understood by reading and understanding the following specification.

In an embodiment, a method includes inputting configuration data into a configuration apparatus, providing a plurality of patient monitoring devices adapted for use in a clinical study, and connecting each of the plurality of patient monitoring devices to the configuration apparatus. The method also includes automatically transmitting the configuration data from the configuration apparatus to each of the plurality of patient monitoring devices such that each of the plurality of patient monitoring devices is generally identically configured in a manner that minimizes labor requirements.

In another embodiment, a method includes inputting configuration data into a configuration apparatus, assembling a patient monitoring device at a manufacturing facility, packaging the patient monitoring device in a packaging material, and connecting the configuration apparatus to the patient monitoring device while the patient monitoring device remains disposed in the packaging material. The method also includes wirelessly transmitting the configuration data from the configuration apparatus to the patient monitoring device while the patient monitoring device remains disposed in the packaging material, and implementing the patient monitoring device to facilitate the performance of a clinical study.

In another embodiment, a system includes a configuration apparatus adapted to receive and retain configuration data, and a plurality of patient monitoring devices that are each independently connectable to the configuration apparatus. The plurality of patient monitoring devices are adapted to facilitate the performance of a clinical study. The configuration apparatus is adapted to selectively transmit the configuration data to each of the plurality of patient monitoring devices such that each of the plurality of patient monitoring devices is generally identically configured in a manner that minimizes labor requirements.

Various other features, objects, and advantages of the invention will be made apparent to those skilled in the art from the accompanying drawings and detailed description thereof.

### BRIEF DESCRIPTION OF THE DRAWINGS

FIGURE 1 is a schematic representation of a system for configuring monitoring devices in accordance with an embodiment;

FIGURE 2 is a flow chart illustrating a method for implementing the system of Figure 1 to configure monitoring devices in a clinical study in accordance with an embodiment; and

FIGURE 3 is a flow chart illustrating a method for implementing the system of Figure 1 to configure monitoring devices in a clinical study in accordance with another embodiment.

### DETAILED DESCRIPTION OF THE INVENTION

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

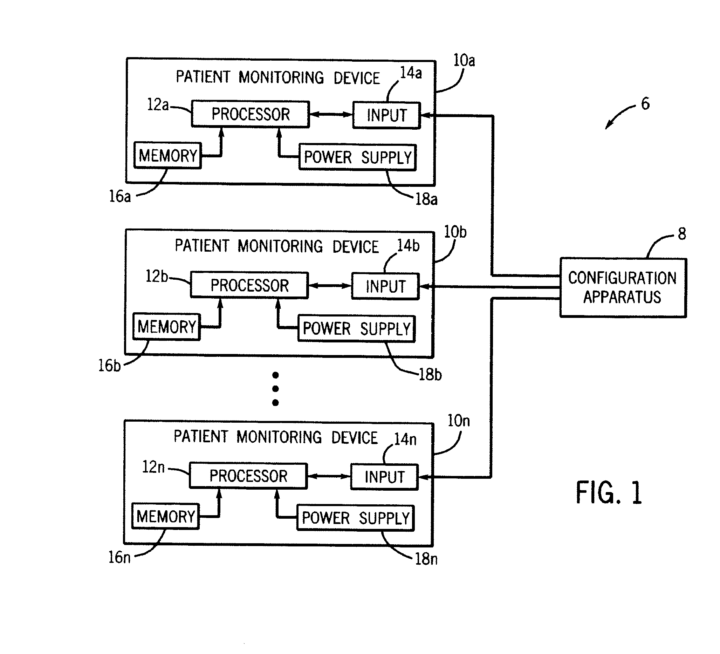

Referring to Figure 1, a system 6 adapted to facilitate the performance of a clinical study is schematically depicted in accordance with an embodiment. For purposes of this disclosure, the term clinical study refers to any research conducted to assess the safety of a medication, device or procedure. The system 6 includes a configuration apparatus 8 that is connectable to each of a plurality of patient monitoring devices 10a-10n.

The configuration apparatus 8 is adapted to at least partially automate the process of configuring the patient monitoring devices 10a-10n. According to one embodiment, the configuration apparatus 8 comprises a programmable computer configured to store and selectively transmit configuration data. For purposes of this disclosure, the term configuration data should be defined to include any data specifying the manner in which the patient monitoring devices 10a-10n are configured and may include, for example, subject identifiers, security modes, filter settings, etc. The patient monitoring devices 10a-10n should be generally identically configured in order to maintain uniformity in the clinical study and to thereby improve the accuracy of the clinical study assessment. Implementing the configuration apparatus 8 to configure each of the patient monitoring devices 10a-10n reduces the manual labor requirements, reduces the likelihood of human error, and improves configuration uniformity.

The patient monitoring devices 10a-10n are adapted for use during the clinical study to provide feedback regarding the safety of a medication, device or procedure. According to one embodiment, the patient monitoring devices 10a-10n may comprise electrocardiograph devices, however other patient monitoring devices may be envisioned. The patient monitoring device 10a will now be described in detail with the understanding that the other patient monitoring devices 10b-10n are structurally and functionally similar. The patient monitoring device 10a includes a processor 12a, an input device 14a, a memory device 16a, and a power supply 18a. The processor 12a is operatively connected to each of the input device 14a, the memory device 16a, and the power supply 18a. The input device 14a is adapted to transfer data to the processor 12a. The memory device 16a is adapted to transfer data to and/or receive data from the processor 12a. The power supply 18a is adapted to power the processor 12a.

The input device 14a may be implemented to transmit configuration data from the configuration apparatus 8 to the processor 12a as the patient monitoring device 10a is being set up for the clinical study. According to one embodiment, the input device 14a comprises a radio frequency identification (RFID) reader or transponder adapted to work in combination with a complementary device disposed in the configuration apparatus 8. According to another embodiment, the input device 14a comprises an Internet connectivity device adapted to work in combination with a similar device disposed in the configuration apparatus 8.

The memory device 16a may be implemented to record configuration data, and to thereafter selectively transmit the recorded configuration data to the processor 12a. The power supply 18a may be implemented to power the processor 12a. According to one embodiment, the power supply 18a comprises a battery system. According to another embodiment, the power supply 18a comprises a solar power system.

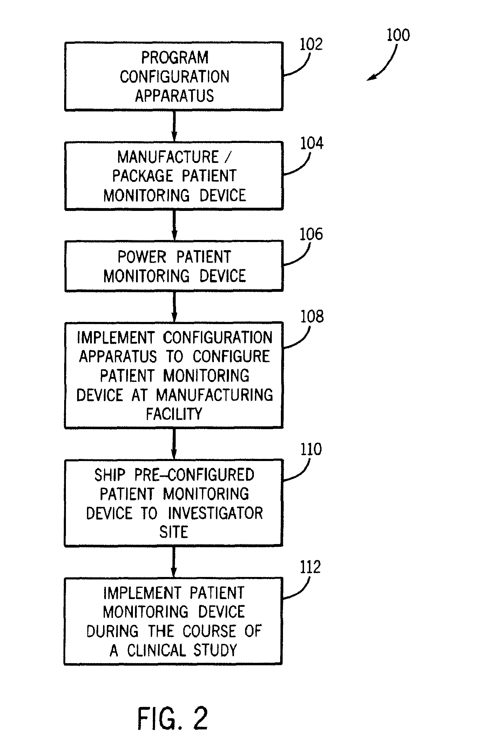

Having described the system 6 in detail, a method 100 for implementing the system 6 to facilitate the performance of a clinical study will now be described with reference to Figures 1 and 2. As shown, the method 100 comprises steps 102-112. The method 100 will hereinafter be described as it applies to the patient monitoring device 10a, however it should be appreciated that the method 100 is also intended for use with the remaining patient monitoring devices 10b-10n.

At step 102, the configuration apparatus 8 is programmed with the configuration data specifying the manner in which each of the patient monitoring devices 10a-10n are to be configured. This step is generally performed by manually inputting the configuration data into the configuration apparatus 8, however it need only be performed one time. In contrast, conventional systems require the manual configuration of each patient monitoring device individually thereby increasing labor requirements and the potential for error.

At step 104, the patient monitoring device 10a is manufactured and packaged at a manufacturing facility. According to one embodiment, at step 104 the patient monitoring device 10a is packaged in a manner adapted to allow it to be powered-up and configured without removing the patient monitoring device 10a from the packaging.

At step 106, the patient monitoring device 10a is powered-up. According to one embodiment, step 106 is automatically performed in response to a signal from the configuration apparatus 8. According to another embodiment, step 106 requires the manual connection of a battery or solar panel.

At step 108, the configuration apparatus 8 is implemented to configure the patient monitoring device 10a. This step is preferably automatically performed at the manufacturing facility by transmitting the configuration data from the configuration apparatus 8 to the input 14a of the patient monitoring device 10a. For purposes of this disclosure, the term automatic refers to a process having one or more automated steps that may be performed without manual intervention. According to one embodiment, the patient monitoring device 10a is configured by wirelessly transmitting the configuration data such as with radio frequency identification (RFID), Bluetooth, or Wi-Fi wireless technology. According to another embodiment, the patient monitoring device 10a is configured by transmitting the configuration data via a cable that is manually plugged into the input 14a.

A specific, non-limiting example of a manner in which step 108 may be performed will now be provided. For purposes of this example, the configuration apparatus 8 includes an RFID transponder and the input 14a comprises an RFID reader. Step 108 may be performed by bringing the patient monitoring device 10a, which may be disposed within packaging material, into close proximity with the configuration apparatus 8. When the patient monitoring device 10a is in sufficiently close proximity, the RFID reader of input 14a will receive a signal from the RFID transponder. This signal may automatically and wirelessly transmit the configuration data from the configuration apparatus 8 to the patient monitoring device 10a. Once transmitted in the manner described, the configuration data can be stored on the memory 16a such that the patient monitoring device 10a is fully configured in accordance with the requirements of the clinical study.

At step 110, the patient monitoring device 10a, which has been pre-configured in the manner described hereinabove, is shipped from the manufacturing facility to the investigator site. For purposes of this disclosure, the investigator site refers to the site at which the patient monitoring device 10a will be implemented during the course of the clinical study. As the patient monitoring device 10a is pre-configured, it may be operated without any additionally programming immediately upon arrival at the investigator site.

At step 112, the patient monitoring device 10a is implemented during the course of a clinical study. According to one embodiment, the patient monitoring device 10a is implemented to monitor clinical study patients and to thereby provide feedback as to the safety of a medication, device or procedure.

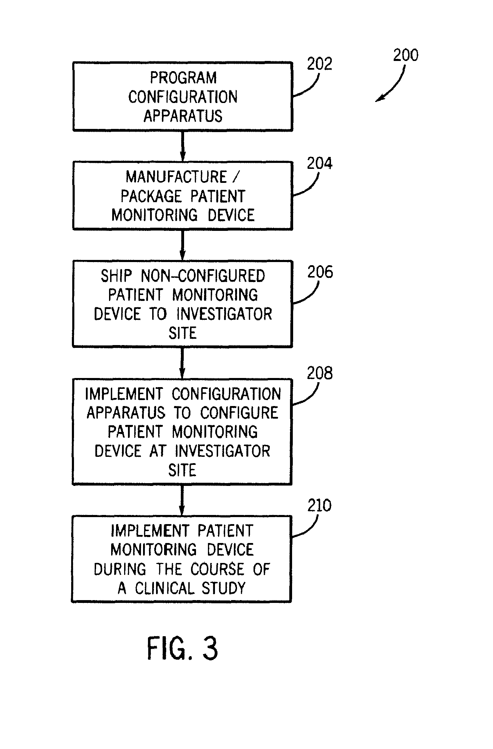

Referring now to Figures 1 and 3, a method 200 for implementing the system 6 to facilitate the performance of a clinical study will now be described in detail. As shown, the method 200 comprises steps 202-210. The method 200 will hereinafter be described as it applies to the patient monitoring device 10a, however it should be appreciated that the method 200 is also intended for use with the remaining patient monitoring devices 10b-10n.

At step 202, the configuration apparatus 8 is programmed with all the configuration data specifying the manner in which each of the patient monitoring devices 10a-10n are to be configured. This step is generally performed by manually inputting the configuration data into the configuration apparatus 8, however it need only be performed one time. In contrast, conventional systems require the manual configuration of each patient monitoring device individually thereby increasing labor requirements and the potential for error.

At step 204, the patient monitoring device 10a is manufactured and packaged at a manufacturing facility. At step 206, the non-configured patient monitoring device 10a is shipped from the manufacturing facility to the investigator site.

At step 208, the configuration apparatus 8 is implemented to configure the patient monitoring device 10a upon arrival at the investigator site. This step is preferably performed by transmitting the configuration data from the remotely located configuration apparatus 8 to the input 14a of the patient monitoring device 10a. According to one embodiment, the patient monitoring device 10a is configured by wirelessly transmitting the configuration data via an Internet connection.

At step 210, the patient monitoring device 10a is implemented during the course of a clinical study. According to one embodiment, the patient monitoring device 10a is implemented to monitor clinical study patients and to thereby provide feedback as to the safety of a medication, device or procedure.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.
Aspects of the present invention are defined in the following numbered clauses:
1. A method comprising:
   inputting configuration data into a configuration apparatus;
   providing a plurality of patient monitoring devices adapted for use in a clinical study;
   connecting each of the plurality of patient monitoring devices to the configuration apparatus; and
   automatically transmitting the configuration data from the configuration apparatus to each of the plurality of patient monitoring devices such that each of the plurality of patient monitoring devices is generally identically configured in a manner that minimizes labor requirements.
2. The method of clause 1, wherein said inputting configuration data into a configuration apparatus comprises manually inputting configuration data into a computer.
3. The method of clause 1, wherein said connecting each of a plurality of patient monitoring devices to the configuration apparatus comprises wirelessly connecting each of the plurality of patient monitoring devices to the configuration apparatus.
4. The method of clause 3, wherein said wirelessly connecting each of the plurality of patient monitoring devices to the configuration apparatus comprises implementing an internet connectivity device to wirelessly connect each of the plurality of patient monitoring devices to the configuration apparatus.
5. The method of clause 3, wherein said wirelessly connecting each of the plurality of patient monitoring devices to the configuration apparatus comprises implementing a radio frequency identification device to wirelessly connect each of the plurality of patient monitoring devices to the configuration apparatus.
6. The method of clause 5, wherein said automatically transmitting the configuration data comprises individually bringing each of said plurality of patient monitoring devices into close proximity with said configuration apparatus such that said radio frequency identification device can be implemented to transmit the configuration data.
7. The method of clause 1, further comprising shipping the plurality of patient monitoring devices to an investigator site before said connecting each of the plurality of patient monitoring devices to the configuration apparatus.
8. The method of clause 1, further comprising shipping the plurality of patient monitoring devices to an investigator site after said automatically transmitting the configuration data such that the plurality of patient monitoring devices are pre-configured upon arrival at the investigator site.
9. The method of clause 1, further comprising implementing the plurality of patient monitoring devices to facilitate the performance of a clinical study after said automatically transmitting the configuration data.
10. A method comprising:
   inputting configuration data into a configuration apparatus;
   assembling a patient monitoring device at a manufacturing facility, said patient monitoring device being adapted for use in a clinical study;
   packaging the patient monitoring device in a packaging material;
   connecting the configuration apparatus to the patient monitoring device while the patient monitoring device remains disposed in the packaging material;
   wirelessly transmitting the configuration data from the configuration apparatus to the patient monitoring device while the patient monitoring device remains disposed in the packaging material; and
   implementing the patient monitoring device to facilitate the performance of a clinical study.
11. The method of clause 10, wherein said inputting configuration data into a configuration apparatus comprises manually inputting configuration data into a computer.
12. The method of clause 10, wherein said connecting the configuration apparatus to the patient monitoring device comprises connecting the configuration apparatus to the patient monitoring device at the manufacturing facility.
13. The method of clause 10, wherein said connecting the configuration apparatus to the patient monitoring device comprises remotely connecting the configuration apparatus to the patient monitoring device via an internet connection after the patient monitoring device reaches an investigator site.
14. A system comprising:
   a configuration apparatus adapted to receive and retain configuration data;
   and
   a plurality of patient monitoring devices that are each independently connectable to the configuration apparatus, said plurality of patient monitoring devices being adapted to facilitate the performance of a clinical study;
   wherein the configuration apparatus is adapted to selectively transmit the configuration data to each of the plurality of patient monitoring devices such that each of the plurality of patient monitoring devices is generally identically configured in a manner that minimizes labor requirements.
15. The system of clause 14, wherein the plurality of patient monitoring devices each comprise a radio frequency identification device.
16. The system of clause 14, wherein the plurality of patient monitoring devices each comprise an internet connectivity device.

## Claims

1. A system (6) comprising:
a configuration apparatus (8) adapted to receive and retain configuration data; and
a plurality of patient monitoring devices (10a-10n) that are each independently connectable to the configuration apparatus (8), said plurality of patient monitoring devices (10a-10n) being adapted to facilitate the performance of a clinical study;
wherein the configuration apparatus (8) is adapted to selectively transmit the configuration data to each of the plurality of patient monitoring devices (10a-10n) such that each of the plurality of patient monitoring devices (10a-10n) is generally identically configured in a manner that minimizes labor requirements.

2. The system (6) of claim 1, wherein the configuration apparatus (8) comprises a programmable computer.

3. The system (6) of claim 1 or claim 2, wherein the plurality of patient monitoring devices (10a-10n) comprise electrocardiograph devices.

4. The system (6) of any one of the preceding claims, wherein the plurality of patient monitoring devices (10a-10n) each include an input (14a-14n) comprising a radio frequency identification device.

5. The system (6) of any one of the preceding claims, wherein the plurality of patient monitoring devices (10a-10n) each include an input (14a-14n) comprising an Internet connectivity device.

6. The system (6) of any one of the preceding claims, wherein the plurality of patient monitoring devices (10a-10n) each comprise a memory (16a-16n) adapted to retain the configuration data from the configuration apparatus (8).

7. The system (6) of any one of the preceding claims, wherein the plurality of patient monitoring devices (10a-10n) each comprise a power supply (18a-18n) configured to automatically provide power in response to a signal from the configuration apparatus (8).

8. The system (6) of any one of the preceding claims, wherein the configuration apparatus (8) is configured to wirelessly transmit the configuration data to each of the plurality of patient monitoring devices (10a-10n).

9. The system (6) of any one of the preceding claims, wherein the configuration apparatus (8) is configured to automatically transmit the configuration data to each of the plurality of patient monitoring devices (10a-10n).

10. A method comprising:
inputting configuration data into a configuration apparatus;
providing a plurality of patient monitoring devices adapted for use in a clinical study;
connecting each of the plurality of patient monitoring devices to the configuration apparatus; and
automatically transmitting the configuration data from the configuration apparatus to each of the plurality of patient monitoring devices such that each of the plurality of patient monitoring devices is generally identically configured in a manner that minimizes labor requirements.
